# EUROPEAN PATENT APPLICATION

(11) **EP 3 778 542 A1**
(43) Date of publication of application: **17.02.2021**
(21) Application number: 19774853.6
(22) Date of filing: 18.03.2019
(51) Int. Cl.: C07C 51/15, C07C 57/04, C07B 61/00

(54) **METHOD FOR PRODUCING ALPHA, BETA-UNSATURATED CARBOXYLATE**

(30) Priority: 28.03.2018 JP 2018061752; 27.12.2018 JP 2018246136
(71) Applicant: SEKISUI CHEMICAL CO., LTD., Osaka-shi Osaka 530-8565 (JP)
(72) Inventor: SHINMEI, Kenichi, Tsukuba-shi, Ibaraki 300-4292 (JP); DASANAYAKE ALUTHGE, Rasika, Tsukuba-shi, Ibaraki 300-4292 (JP); MIYAMA, Toshihito, Tsukuba-shi, Ibaraki 300-4292 (JP)
(74) Representative: Ter Meer Steinmeister & Partner
(86) International application number: PCT/JP2019/011206
(87) International publication number: WO 2019/188507

(57) **Abstract**

A method for producing an α,β-unsaturated carboxylic acid salt, including: a step (1) of reacting a transition metal complex, an alkene and carbon dioxide to obtain a metal lactone compound represented by formula (1): 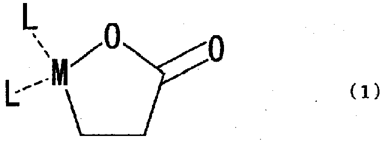 wherein: M is a transition metal, each L is independently a monodentate ligand, or two L together form a bidentate ligand; and a step (2) of allowing a base to act on the metal lactone compound, wherein a molar amount (A) of the carbon dioxide per mol of the transition metal complex in the step (1) is 0.1 to 10 mol.

## Description

### TECHNICAL FIELD

The present invention relates to a method for producing an α,β-unsaturated carboxylic acid salt.

Priorities are claimed on Japanese Patent Application No. 2018-061752, filed March 28, 2018, and Japanese Patent Application No. 2018-246136, filed December 27, 2018, the contents of which are incorporated herein by reference.

### BACKGROUND ART

In recent years, for reducing greenhouse gases such as carbon dioxide (CO₂) or other purposes, many methods have been proposed with respect to the chemical synthesis using CO₂ as a raw material. As one of such methods, a production of unsaturated carboxylic acids such as acrylic acid using CO₂ and an alkene as a raw materials is proposed.

For example, Non-Patent Document 1 describes the world's first catalytic synthesis of acrylic acid salts from CO₂ and an alkene. More specifically, a nickel complex is reacted with ethylene to obtain an ethylene-nickel complex (step 1), which is then reacted with CO₂ to form a nickel-lactone complex (step 2). Further, it is described that sodium tert-butoxide was added and reacted with the nickel-lactone complex to release the acrylate (sodium acrylate) via the formation of the acrylate complex by cleavage of the lactone ring and substitution of the acrylate ligand by ethylene.

The Non-Patent Document 1 describes that CO₂ is reacted while increasing the pressure to 50 bar (about 50 atm) for forming the nickel-lactone complex in the step 2 described above. This document also describes that the post-process following the step 2 is performed after the CO₂-rich region has been changed into the CO₂-poor region by substitution with ethylene.

### Description of Prior Art

### Non-Patent Document

Non-Patent Document 1 : Lejkowski, M. L. et al., "The first catalytic synthesis of an acrylate from CO2 and an alkene-A rational approach", Chem. Eur. J. 18, 14017-14025 (2012)

### DISCLOSURE OF INVENTION

### Problems to be Solved by the Invention

According to Non-Patent Document 1, an α,β-unsaturated carboxylic acid salt, such as acrylates, can be catalytically synthesized from CO₂ and ethylene.

However, first of all, in the step 2, CO₂ is pressurized to 50 bar (about 50 atm) to form the nickel-lactone complex. This is not efficient as an extremely excessive amount of CO₂ must be used with respect to the ethylene-nickel complex, which is the raw material for the nickel-lactone complex. Further, this process is inefficient also in that the excessive amount of CO₂ is subsequently discharged by the ethylene substitution.

In this context, the object of the present invention is to provide a means for efficiently producing an α,β-unsaturated carboxylic acid salt.

### Means to Solve the Problems

The present inventors have made intensive studies to solve the above problems. As a result, they have found that the above problems can be solved by separating the production process into a step of obtaining a metal lactone compound and a step of allowing a base to act on the metal lactone compound as well as limiting the amount of CO₂ to fall within a specific range. Based on this finding, the present invention has been completed.

Specifically, the present invention has the following configurations.
[1] A method for producing an α,β-unsaturated carboxylic acid salt, including:
   a step (1) of reacting a transition metal complex, an alkene and carbon dioxide to obtain a metal lactone compound represented by formula (1): 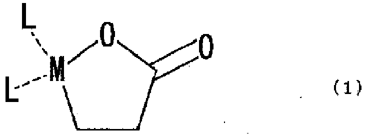 wherein:
      M is a transition metal,
      each L is independently a monodentate ligand, or two L together form a bidentate ligand; and
   a step (2) of allowing a base to act on the metal lactone compound,
      wherein a molar amount (A) of the carbon dioxide per mol of the transition metal complex in the step (1) is 0.1 to 10 mol.
[2] The method according to [1], wherein the metal lactone compound is represented by formula (2): 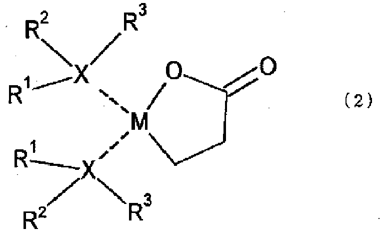 wherein:
   M is a transition metal,
   each X is independently a nitrogen atom or a phosphorus atom,
   each R¹ is independently an aliphatic hydrocarbon group, an aromatic hydrocarbon group, a heteroaromatic group, or a nitrogen-containing group, and
   each of R² and R³ is independently an aliphatic hydrocarbon group, an aromatic hydrocarbon group, or a heteroaromatic group,
   with the proviso that two R¹ are or are not bonded to each other to form a ring structure, and
   R¹, R², and R³, which are bonded to the same X atom, are or are not bonded together to form a ring structure.
[3] The method according to [2], wherein the metal lactone compound is represented by formula (3): 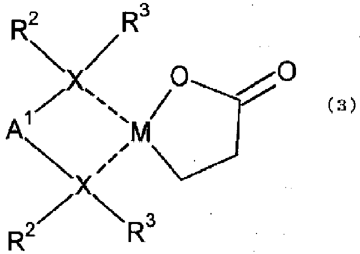 wherein:
   M is a transition metal,
   each X is independently a nitrogen atom or a phosphorus atom,
   each of R² and R³ is independently an aliphatic hydrocarbon group, an aromatic hydrocarbon group, or a heteroaromatic group, and
   A¹ is a single bond, a divalent aliphatic hydrocarbon group, a divalent aromatic hydrocarbon group, a divalent heteroaromatic group or a nitrogen-containing group,
   with the proviso that A¹, R², and R³, which are bonded to the same X atom, are or are not bonded together to form a ring structure.
[4] The method according to [3], wherein at least one of the X is a nitrogen atom, and
   A¹, R², and R³, which are bonded to the nitrogen atom, are bonded together to form a heteroaromatic ring.
[5] The method according to [3] or [4], wherein at least one of the X is a phosphorus atom, and
   at least one of R² and R³, which are bonded to the phosphorus atom, is an aliphatic hydrocarbon group having 3 or more carbon atoms, an aromatic hydrocarbon group having 6 or more carbon atoms, or a heteroaromatic group having 3 or more carbon atoms.
[6] The method according to any one of [3] to [5], wherein the metal lactone compound is at least one compound represented by formulae (4) to (6): 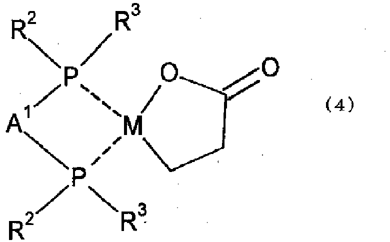 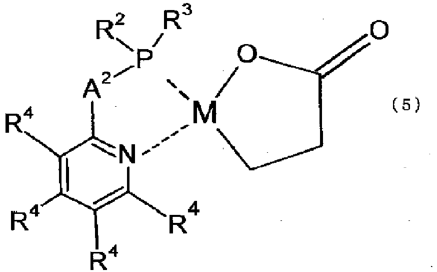 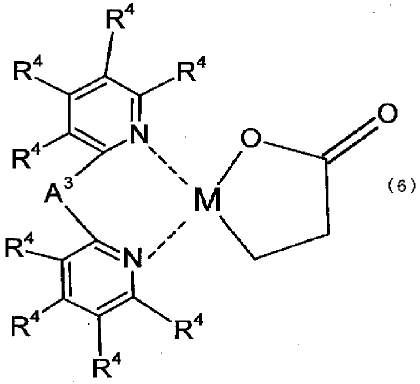 wherein:
   M is a transition metal,
   each of R² and R³ is independently an aliphatic hydrocarbon group, an aromatic hydrocarbon group, or a heteroaromatic group,
   A¹ is a single bond, a divalent aliphatic hydrocarbon group, a divalent aromatic hydrocarbon group, a divalent heteroaromatic group or a nitrogen-containing group,
   A² is a single bond, a divalent aliphatic hydrocarbon group, a divalent aromatic hydrocarbon group, a divalent heteroaromatic group or a nitrogen-containing group,
   A³ is a single bond, a divalent aliphatic hydrocarbon group, a divalent aromatic hydrocarbon group, a divalent heteroaromatic group or a nitrogen-containing group, and
   each R⁴ is independently a hydrogen atom, an aliphatic hydrocarbon group, an aromatic hydrocarbon group, or a heteroaromatic group.
[7] The method according to any one of [1] to [6], wherein a molar amount (B) of the base per mol of the transition metal complex in the step (2) is not more than the molar amount (A) of the carbon dioxide.
[8] The method according to any one of [1] to [7], wherein the reaction in the step (1) is carried out at a pressure of 10 atm or less.
[9] The method according to any one of [1] to [8], wherein the step (1) and the step (2) are alternately repeated.

### Effect of the Invention

According to the present invention, an α,β-unsaturated carboxylic acid salt can be efficiently produced.

### DESCRIPTION OF THE EMBODIMENTS

Hereinbelow, the present invention will be described in detail.

The method of the present invention for producing an α,β-unsaturated carboxylic acid salt includes: a step (1) of reacting a transition metal complex, an alkene and carbon dioxide (CO₂) to obtain a metal lactone compound represented by formula (1) below; and a step (2) of allowing a base to act on the metal lactone compound.

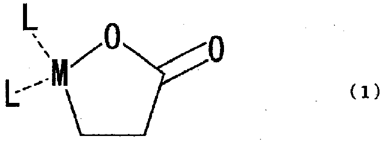

In the formula (1), M is a transition metal, and each L is independently a monodentate ligand, or two L together form a bidentate ligand. In the step (1), the molar amount (A) of the carbon dioxide per 1 mol of the transition metal complex is 0.1 to 10 mol.

This enables production of an α,β-unsaturated carboxylic acid salt with high reaction efficiency. Specifically, by restricting the molar amount (A) of carbon dioxide used in the step (1) to fall within a range of 0.1 to 10 mol per mol of the transition metal complex, all or most of the carbon dioxide is allowed to be consumed in the step (1) to thereby increase the reaction efficiency.

### [Step (1)]

In step (1), the transition metal complex, the alkene and carbon dioxide are reacted to obtain a metal lactone compound represented by the formula (1).

### (Transition Metal Complex)

The transition metal complex generally contains a transition metal and a ligand. Examples of the transition metal include, but are not particularly limited to, elements belonging to group 6 of the periodic table, such as chromium (Cr), molybdenum (Mo), and tungsten (W); elements belonging to group 7 of the periodic table, such as rhenium (Re); elements belonging to group 8 of the periodic table, such as iron (Fe) and ruthenium (Ru); elements belonging to group 9 of the periodic table, such as cobalt (Co) and rhodium (Rh); and elements belonging to group 10 of the periodic table, such as nickel (Ni), palladium (Pd), and platinum (Pt). Among these, as the transition metal, nickel, molybdenum, cobalt, iron, iron, rhodium, ruthenium, palladium, platinum, rhenium, and tungsten are preferable; nickel, molybdenum, palladium, platinum, cobalt, iron, rhodium, and ruthenium are more preferable; and nickel and palladium are even more preferable. One of these transition metals may be used alone or two or more of these may be used in combination.

The ligand(s) may be a monodentate ligand or a multidentate ligand such as a bidentate ligand, a tridentate ligand or the like, but it is desirable to select an appropriate ligand so as to leave coordination site for CO₂ and a compound to be reacted therewith on the metal. For example, when the active metal is nickel, it is preferable to use a bidentate ligand, and when the active metal is cobalt, it is preferable to use a tridentate ligand.

The ligand may contain at least one atom or atomic group selected from the group consisting of a phosphorus atom, a nitrogen atom, an oxygen atom, and a carbene group as an atom or atomic group coordinating to the transition metal. The ligand(s) can be selected from, for example, phosphines, phosphites, amines, and N-heterocyclic carbenes. In the ligand, the aforementioned "at least one atom or atomic group coordinating to the transition metal" is preferably at least one atom or atomic group selected from the group consisting of a phosphorus atom, an amine and a carbene group, and more preferably a phosphorus atom and/or an amine.

When the ligand contains at least one phosphorus atom coordinating to the transition metal, it is preferred that at least one group is attached to the phosphorus atom via a secondary or tertiary carbon atom. More preferably, at least two groups are bonded to the phosphorus atom via a secondary or tertiary carbon atom. Examples of suitable groups bonded to the phosphorus atom via a secondary or tertiary carbon atom include adamantyl, tert-butyl, cyclohexyl, sec-butyl, isopropyl, phenyl, tolyl, xylyl, mesityl, naphthyl, fluorenyl, and anthracenyl. Among them, a group having a high electron donating property is desirable. Specifically, tert-butyl and cyclohexyl are preferable.

When the ligand has at least one N-heterocyclic carbene coordinating to the transition metal, it is preferable that at least one group is bonded via at least one secondary or tertiary carbon atom to at least one α-nitrogen atom of the carbene group. Examples of suitable groups bonded to the nitrogen atom via a tertiary carbon atom include adamantyl, tert-butyl, isopropyl, phenyl or 2,6-diisopropylphenyl. Among these, adamantyl, tert-butyl and 2,6-diisopropylphenyl are preferable.

With respect to the ligand containing at least one phosphorus atom that can be used in the present invention, specific examples of the monodentate ligand include triarylphosphines such as trimethylphosphine; tricycloalkylphosphines such as tricyclohexylphosphine (PCy3); triarylphosphines such as triphenylphosphine, and tri(4-fluoromethylphenyl)phosphine; triheteroarylphosphines such as tri-2-furanylphosphine; and phosphorane ligands such as triphenylphosphine oxide.

Specific examples of the bidentate ligand include bis(diphenylphosphino)methane (dppm), 1,2-bis(diphenylphosphino)ethane (dppe), 1,3-bis(diphenylphosphino)propane (dppp), 1,4-bis(diphenylphosphino)butane (dppb), 1,5-bis(diphenylphosphino)pentane (dpppe), 1,6-bis(diphenylphosphino)hexane, 1,1'-bis(diphenylphosphino)ferrocene, 1,2-bis(dipentafluorophenylphosphino)ethane, 1,2-bis(dicyclohexylphosphino)ethane, 1,3-bis(dicyclohexylphosphino)propane, 1,4-bis(dicyclohexylphosphino)butane, 1,2-bis(di-tert-butylphosphino)ethane, 1,2-bis(diphenylphosphino)benzene, 1,2-bis(bis(3,5-dimethylphenyl)phosphino)ethane, 1,3-bis(bis(3,5-dimethylphenyl)phosphino)propane, 1,4-bis(bis(3,5-dimethylphenyl)phosphino butane, 1,2-bis(cyclohexylphosphino)ethane, 1,4-bis(bis(3,5-di-tert-butylphenyl)phosphino)butane, 1,4-bis(bis(3,5-dimethoxyphenyl)phosphino)butane, 2,2'-bis(diphenylphosphino) -1,1'-binaphthyl, 2,2'-bis(bis(3,5-dimethylphenyl)phosphino) -1,1'-binaphthyl, (2S,3S)-(-)-bis(diphenylphosphino)butane, (S,S) -1,2-bis[(2-methoxyphenyl)phenylphosphino]ethane ((S,S)-DIPAMP), (R,R)-(-)-2,3-bis(tert-butylmethylphosphino)quinoxaline (Quinox P*), (R,R)-1,2-bis(tert-butylmethylphosphino)benzene (BenzP*), 1,1,1-tris(diphenylphosphinomethyl)ethane, 1,1,1-tris (bis(3,5-dimethylphenyl)phosphinomethyl)ethane, 1,1,1-tris (diphenylphosphino)methane, tris(2-diphenylphosphinoethyl) phosphine, (oxydi-2,1-phenylene)bis(diphenylphosphine), and 4,5-bis(diphenylphosphino)-9,9-dimethylxanthene, and 4,5-bis(diphenylphosphino)-9,9-dimethylxanthene.

Specific examples of the tridentate ligand include bis(2-diphenylphosphinoethyl)phenylphosphine, 1,1,1-tris(diphenylphosphinomethyl)ethane. Specific examples of the tetradentate ligand include tris(2-diphenylphosphinoethyl)phenyl phosphine.

Among these, 1,2-bis(diphenylphosphino)ethane (dppe), 1,2-bis(dicyclohexylphosphino)ethane, 1,2-bis(di-tert-butyl phosphino) ethane, 1,3-bis(dicyclohexylphosphino)propane, and BenzP* are preferable.

As the ligand, besides those mentioned above, the transition metal complex may include at least one further ligand selected from the group consisting of halides, amines, amides, oxides, phosphides, carboxylato, acetylacetonato, aryl sulfonatos, alkyl sulfonatos, hydrides, carbon monoxide, alkenes (ethylene, propene, butane, etc.), dienes (1,3-butadiene, 1,6-hexadiene, etc.), cycloalkenes (cyclohexene, etc.), cycloalkadienes (1,5-cyclooctadiene (COD), etc.), nitriles, aromatic compounds, heteroaromatic compounds, ethers, phosphorus trifluoride, phosphole, and phosphabenzene, as well as monodentate, bidentate and polydentate forms of a phosphinite ligand, a phosphonite ligand, a phosphoramidite ligand and a phosphite ligand.

One of the ligands described above may be used alone, or two or more thereof may be used in combination.

Specific examples of the transition metal complex used in one embodiment include those represented by the following formula (7).

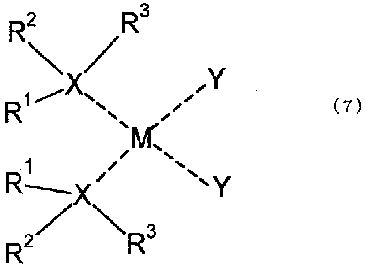

M is a transition metal, and the specific examples thereof are as described above. Among those, a nickel atom and a palladium atom are preferable as M.

Each X is independently a nitrogen atom or a phosphorus atom.

Y is carbon monoxide, an alkene having 2 to 20 carbon atoms, a diene having 4 to 20 carbon atoms, a cycloalkene having 3 to 20 carbon atoms, or a cycloalkadiene having 4 to 20 carbon atoms.

Examples of the alkene having 2 to 20 carbon atoms include, but are not particularly limited to, ethylene, propylene, isobutene, 1,3-butadiene, piperylene, 1-butene, 2-butene, 1-pentene, 3-pentene, 1-hexene, 1-heptene, 1-octene, 1-nonene, 1-decene and the like.

Examples of the diene having 4 to 20 carbon atoms include, but are not particularly limited to, 1,3-butadiene, 1,3-pentadiene, 1,4-pentadiene, 1,4-hexadiene, 1,5-hexadiene, 2-methyl-1,3-butadiene, and the like.

Examples of the cycloalkene having 3 to 20 carbon atoms include, but are not particularly limited to, cyclopropene, cyclobutene, cyclopentene, cyclohexene, methylcyclopentene, ethylcyclopentene, cyclohexene, and the like.

Examples of the cycloalkadiene having 4 to 20 carbon atoms includes 1,5-cyclooctadiene (COD).

Of the examples described above, Y is preferably carbon monoxide, an alkene having 2 to 20 carbon atoms or a cycloalkadiene having 4 to 20 carbon atoms, more preferably carbon monoxide, an alkene having 2 to 10 carbon atoms or a cycloalkadiene having 4 to 10 carbon atoms, even more preferably carbon monoxide, ethylene or 1,5-cyclooctadiene (COD), and especially preferably ethylene or 1,5-cyclooctadiene (COD).

Each R¹ is independently an aliphatic hydrocarbon group, an aromatic hydrocarbon group, a heteroaromatic group, or a nitrogen-containing group. Further, each of R² and R³ is independently an aliphatic hydrocarbon group, an aromatic hydrocarbon group, or a heteroaromatic group. In this context, two R¹ may be bonded to each other to form a ring structure, and R¹, R², and R³, which are bonded to the same X atom, may be bonded together to form a ring structure.

Examples of the aliphatic hydrocarbon group include alkyl groups having 2 to 20 carbon atoms, cycloalkyl groups having 3 to 20 carbon atoms, and the like.

Examples of the alkyl group having 2 to 20 carbon atoms include, but are not particularly limited to, a methyl group, an ethyl group, a propyl group, an isopropyl group, an n-butyl group, a 2-butyl group, a sec-butyl group, a tert-butyl group, an n-pentyl group, a 2-pentyl group, 2-methylbutyl group, 3-methylbutyl group, 1,2-dimethylpropyl group, a 1,1-dimethylpropyl group, a 2,2-dimethylpropyl group, a 1-ethylpropyl group, a n-hexyl group, a 2-hexyl group, a 2-methylpentyl group, a 3-methylpentyl group, a 4-methylpentyl group, a 1,2-dimethylbutyl group, a 1,3-dimethylbutyl group, a 2,3-dimethylbutyl group, a 1,1-dimethylbutyl group, a 2,2-dimethylbutyl group, a 3,3-dimethylbutyl group, a 1,1,2-trimethylpropyl group, a 1,2,2-trimethylpropyl group, a 1-ethylbutyl group, a 2-ethylbutyl group, 1-ethyl-2-methylpropyl group, a n-heptyl group, a 2-heptyl group, a 3-heptyl group, a 2-ethylpentyl group, a 1-propylbutyl group, a n-octyl group, a 2-ethylhexyl group, a 2-propylheptyl group, a nonyl group, and a decyl group.

Examples of the cycloalkyl group having 3 to 20 carbon atoms include, but are not particularly limited to, a cyclopropyl group, a cyclobutyl group, a cyclopentyl group, a cyclohexyl group, a cycloheptyl group, a cyclooctyl group, and an adamantyl group.

In this context, the aliphatic hydrocarbon group may have a substituent. Examples of the substituent include alkoxy groups (e.g., a methoxy group, an ethoxy group, a propoxy group, etc.), aryl groups (e.g., a phenyl group, etc.), heteroaryl groups (e.g., a pyridinyl group), a hydroxy group, halogen atoms (e.g., a fluorine atom, a chlorine atom, a bromine atom, etc.), a nitro group, a nitrile group, NE¹E² groups, NE¹E²E³⁺ groups, carboxylic acid derivative groups (e.g., carboxylic acid ester groups such as a methoxycarbonyl group, etc.), sulfonic acid derivative groups (e.g., sulfonic acid ester groups, sulfonamide groups, etc.). In this context, each of E¹, E² and E³ is independently a hydrogen atom, an alkyl group having 2 to 20 carbon atoms, a cycloalkyl group having 3 to 20 carbon atoms, or an aryl group having 6 to 30 carbon atoms. One of these substituents may be present alone, or two or more of these may be present in combination.

Examples of the aromatic hydrocarbon group include aryl groups having 6 to 30 carbon atoms. Examples of the aryl group having 6 to 30 carbon atoms include a phenyl group, a naphthyl group, an anthryl group, a phenanthryl group, and a biphenyl group.

Examples of the heteroaromatic group include heteroaryl groups having 3 to 30 carbon atoms. Examples of the heteroaryl group having 3 to 30 carbon atoms include a furanyl group, a thiophenyl group, a pyrrolyl group, a pyrazolyl group, an imidazolyl group, an isoxazolyl group, a thiazolyl group, a thiadiazolyl group, and a pyridyl group, a pyridazinyl group, a pyrimidinyl group, a pyrazinyl group, a triazinyl group, a benzofuranyl group, an indolyl group, a thianaphthenyl group, a benzimidazolyl group, and a benzoxazolyl, a benzothiazolyl, a benzotriazolyl group, a quinolyl group, an isoquinolyl group, a tinolyl group, a quinoxalyl group, a dibenzothiophenyl group, an acridyl group, and a phenanthryl group.

Each of the aromatic hydrocarbon group and the heteroaromatic group may have a substituent. Examples of the substituent include, but are not limited to, an alkyl group having 2 to 20 carbon atoms, a cycloalkyl group having 3 to 20 carbon atoms, and an alkoxy group, a hydroxy group, a halogen atom, a nitro group, a nitrile group, an NE¹E² group, an NE¹E²E³⁺ group, a carboxy group, a sulfo group, and the like.

The nitrogen-containing group is different from the heteroaromatic ring group, and examples thereof include the NE¹E² group and the NE¹E²E³⁺ group. E¹, E², and E³ are as described above. Specific examples of the NE¹E² group and the NE¹E²E³⁺ group include an amino group, a methylamino group, an ethylamino group, a dimethylamino group, and a diethylamino group.

In this context, the phrase "two R¹ are .....bonded to each other to form a ring structure" means that two R¹ are bond to each other, resulting in a divalent group being formed by these two R¹ groups to form a ring structure together with two X and M. Further, regarding the phrase "R¹, R², and R³, which are bonded to the same X atom, are ..... bonded together to form a ring structure", this typically means that that R¹, R², and R³, which are bonded to the same X atom, together form a ring structure containing a double bond. As an example of such a case, R¹ and R² together with X form a 6-membered ring structure, while a double bond in the 6-membered ring structure is formed at the bonding site of R³.

The aforementioned two R¹ are preferably bonded to each other to form a ring structure, more preferably a three-membered, 4-membered, 5-membered or 6-membered ring structure, even more preferably a 5-menbered or 6-membered ring structure. The formation of a ring structure by mutual bonding between the two R¹ is favorable in that the two R¹ form a bidentate ligand and makes the complex chemically stable, and the ring structure restricts the conformation between the metal and the bidentate ligand and distorts the lactone ring structure upon formation of the metal lactone compound to thereby facilitate the cleavage of the lactone ring in the step (2).

Each of R² and R³ is preferably an alkyl group having 1 to 20 carbon atoms, or a cycloalkyl or heteroaromatic group having 3 to 20 carbon atoms, more preferably a cycloalkyl group, an aromatic hydrocarbon group, or a heteroaromatic group, each having 6 to 20 carbon atoms, and even more preferably an alkyl group having 3 to 10 carbon atoms or a cycloalkyl group having 6 to 20 carbon atoms. Each of R² and R³ is preferably an alkyl group having 3 to 10 carbon atoms, or a cycloalkyl, aromatic hydrocarbon or heteroaromatic group having 3 to 20 carbon atoms, because these groups have large steric hindrance and have electron-donating capacity, so that the complex forming reaction at Y is facilitated.

In one embodiment, the transition metal complex is preferably one represented by the following formula (8).

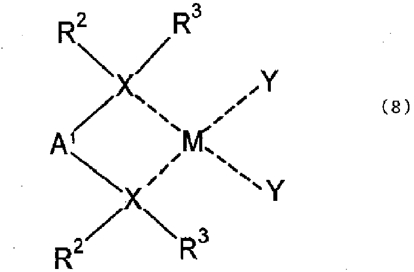

That is, this shows a structure of the formula (7) above, in which the two R¹ are bonded to each other to form a ring structure.

As for M, X, and Y, the same applies as described above for the formula (7).

As for R² and R³, the same applies as described above for the formula (7).

A¹ is a single bond, a divalent aliphatic hydrocarbon group, a divalent aromatic hydrocarbon group, a divalent heteroaromatic group or a nitrogen-containing group. In this context, A¹, R², and R³, which are bonded to the same X atom, may be bonded together to form a ring structure.

Examples of the divalent aliphatic hydrocarbon group include alkylene groups having 1 to 20 carbon atoms, cycloalkylene groups having 3 to 20 carbon atoms, and alkenylene groups having 2 to 20 carbon atoms.

Examples of the alkylene group having 1 to 20 carbon atoms include a methylene group, an ethylene group, a propylene group, an isopropylene group, a butylene group, a 2-methylpropylene group, a pentylene group, and the like.

Examples of the cycloalkylene group having 3 to 20 carbon atoms include a cyclopropylene group, a cyclobutylene group, a cyclopentylene group, a cyclohexylene group, a methylcyclohexylene group, and the like.

Examples of the alkenylene groups having 2 to 20 carbon atoms include a vinylene group, a propenylene group, and a butenylene group.

The divalent aliphatic hydrocarbon group may have a substituent. Examples of the substituent include the same as those mentioned above for the aliphatic hydrocarbon group. The divalent aliphatic hydrocarbon group may have one substituent alone or two or more substituents in combination.

Examples of the divalent aromatic hydrocarbon group include divalent groups derived from arylenes having 6 to 30 carbon atoms. Examples of the arylene group having 6 to 30 carbon atoms include divalent groups derived from benzene, toluene, xylene, xylene, naphthalene, biphenyl, and terphenyl.

Examples of the divalent heterocyclic aromatic compound group includes divalent groups derived from heteroarylenes having 1 to 30 carbon atoms. Examples of the heteroarylene group having 1 to 30 carbon atoms include divalent groups derived from furan, thiophene, pyrrole, pyrazole, imidazole, isoxazole, thiazole, thiadiazole, pyridine, pyridazine, pyrimidine, pyrazine, triazine, benzofuran, indole, thianaphthene, benzimidazole, benzoxazole, benzothiazole, benzotriazole, purine, quinoline, isoquinoline, tinoline, quinoxaline, dibenzothiophene, acridine, and phenanthroline.

Each of the divalent aromatic hydrocarbon group and the divalent heteroaromatic group may have a substituent. Examples of the substituent include those mentioned above for the aromatic hydrocarbon group and the heteroaromatic group.

Each of the divalent aromatic hydrocarbon and the divalent heteroaromatic group may have one substituent alone or two or more substituents in combination.

Examples of the nitrogen-containing group include -NE⁴-. In this -NE⁴-, E⁴ is a hydrogen atom, an alkyl group having 2 to 20 carbon atoms, a cycloalkyl group having 3 to 20 carbon atoms, an aryl group having 6 to 30 carbon atoms, or a heteroaryl group having 3 to 30 carbon atoms.

Regarding the phrase "A¹, R², and R³, which are bonded to the same X atom, are..... bonded together to form a ring structure", this typically means that that A¹, R², and R³, which are bonded to the same X atom, together form a ring structure containing a double bond.

In the formula (8) above, at least one of the X atoms is preferably a nitrogen atom. In this case, the transition metal complex of the formula (8) preferably includes a heteroaromatic ring formed by mutual bond between A¹, R², and R³, which are bonded to the nitrogen atom. Examples of the heteroaromatic ring include a pyrrole ring, a pyrazole ring, an imidazole ring, a pyridine ring, a pyridazine ring, a pyrimidine ring, a pyrazine ring, a triazine ring, and the like. Among these, a pyridine ring, a pyridazine ring, a pyrimidine ring, a pyrazine ring, and a triazine ring are preferable, and a pyridine ring is more preferable. In this context, the heteroaromatic ring may have a substituent group. Examples of the substituent include an alkyl group having 2 to 20 carbon atoms, a cycloalkyl group having 3 to 20 carbon atoms, and an alkoxy group, a hydroxy group, a halogen atom, a nitro group, a nitrile group, an NE¹E² group, an NE¹E²E³⁺ group, a carboxy group, a sulfo group, and the like. One of these substituents may be present alone, or two or more of these may be present in combination. The heteroaromatic ring possessed by the transition metal complex of the formula (8) shows a certain level of steric hindrances and has a high electron-donating capacity attributable to a heteroatom such as a nitrogen atom, which is favorable in that the complex forming reaction at Y is facilitated.

In the formulae (7) and (8), it is preferable that at least one of the X atoms is a phosphorus atom, and at least one of R² and R³, which are bonded to the phosphorus atom, is an aliphatic hydrocarbon group having 3 or more carbon atoms, an aromatic hydrocarbon group having 6 or more carbon atoms, or a heteroaromatic group having 3 or more carbon atoms.

Examples of the aliphatic hydrocarbon group having 3 or more carbon atoms include alkyl groups having 3 to 20 carbon atoms, such as a propyl group, an isopropyl group, a butyl group, an isobutyl group, a sec-butyl group, a tert-butyl group, a pentyl group, an isopentyl group, a 2-ethylbutyl group, a hexyl group, a 2-methylpentyl group, a 3-methylpentyl group, a heptyl group, an a 2-methylhexyl group; and cycloalkyl groups having 3 to 20 carbon atoms, such as a cyclopropyl group, a cyclobutyl group, a cyclopentyl group, a cyclohexyl group, and cycloheptyl group. In this context, the aliphatic hydrocarbon group having 3 or more carbon atoms may have a substituent. Examples of the substituent include an alkoxy group, an aryl group, a heteroaryl groups, a hydroxy group, a halogen atom, a nitro group, a nitrile group, an NE¹E² group, a NE¹E²E²E³⁺ group, a carboxylic acid derivative group, a sulfonic acid derivative group, and the like.

One of these substituents may be present alone, or two or more of these may be present in combination.

Examples of the aromatic hydrocarbon group having 6 or more carbon atoms include heteroaryl groups having 6 to 30 carbon atoms, such as a phenyl group, a naphthyl group, an anthryl group, a phenanthryl group and a biphenyl group.

Examples of the heteroaromatic group having 3 or more carbon atoms include heteroaryl groups having 3 to 30 carbon atoms, such as a furanyl group, a thiophenyl group, a pyrrolyl group, a pyrazolyl group, an imidazolyl group, an isoxazolyl group, a thiazolyl group, a thiadiazolyl group, and a pyridyl group, a pyridazinyl group, a pyrimidinyl group, a pyrazinyl group, a triazinyl group, a benzofuranyl group, an indolyl group, a thianaphthenyl group, a benzimidazolyl group, and a benzoxazolyl, a benzothiazolyl group, a benzotriazolyl group, a quinolyl group, an isoquinolyl group, a tinolyl group, a quinoxalyl group, a dibenzothiophenyl group, an acridyl group, and a phenanthryl group.

Each of the aromatic hydrocarbon group and the heteroaromatic group that have 6 or more carbon atoms may have a substituent. Examples of the substituent include an alkyl group having 2 to 20 carbon atoms, a cycloalkyl group having 3 to 20 carbon atoms, and an alkoxy group, a hydroxy group, a halogen atom, a nitro group, a nitrile group, an NE¹E² group, an NE¹E²E³⁺ group, a carboxy group, a sulfo group, and the like. One of these substituents may be present alone, or two or more of these may be present in combination.

In this context, in the formulae (7) and (8), when at least one of the X atoms is a phosphorus atom, at least one of R² and R³, which are bonded to the phosphorus atom, is preferably an alkyl group having 4 to 8 carbon atoms, a cycloalkyl group having 4 to 8 carbon atoms, an aryl group having 6 to 10 carbon atoms, or a heteroaryl group having 3 to 7 carbon atoms, more preferably an alkyl group having 4 to 8 carbon atoms, a cycloalkyl group having 4 to 8 carbon atoms, or an aryl group having 6 to 10 carbon atoms, and even more preferably an alkyl having 4 to 8 carbon atoms, or a cycloalkyl group having 4 to 8 carbon atoms, and particularly preferably a cycloalkyl group having 4 to 8 carbon atoms.

In one embodiment, the transition metal complex is preferably one represented by the following formula (9), (10) or (11).

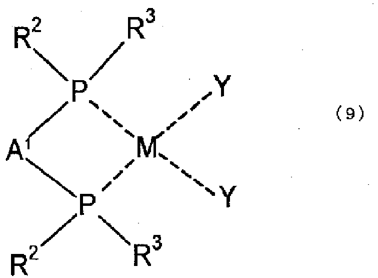

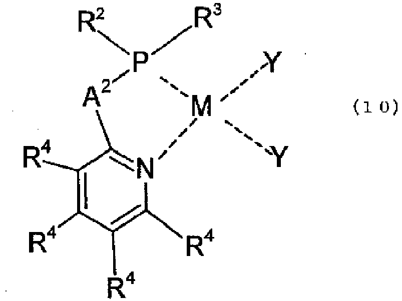

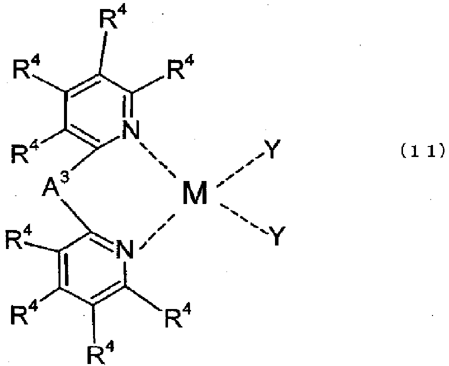

Specifically, the formula (9) represents a structure of the formula (7) in which the two X atoms are phosphorus atoms (P).

The formula (10) represents a structure of the formula (7) in which one of the X atoms is a phosphorus atom while another one of the X atoms is a nitrogen atom, and R¹, R², and R³, which are bonded to the nitrogen atom, together form a pyridine ring. The formula (11) represents a structure of the formula (7) in which two X atoms are nitrogen atoms, and R¹, R², and R³, which are bonded to the nitrogen atoms, together form pyridine rings.

As for M, X, and Y, the same applies as described above for the formula (7).

As for A ¹, the same applies as described above for the formula (8).

A² is a single bond, a divalent aliphatic hydrocarbon group, a divalent aromatic hydrocarbon group, a divalent heteroaromatic group or a nitrogen-containing group. Usually, A² is capable of having a carbon number of: carbon number of A¹ - 1.

A³ is a single bond, a divalent aliphatic hydrocarbon group, a divalent aromatic hydrocarbon group, a divalent heteroaromatic group or a nitrogen-containing group. Usually, A³ is capable of having a carbon number of: carbon number of A¹ - 2.

Each R⁴ is independently a hydrogen atom, an alkyl group having 2 to 20 carbon atoms, a cycloalkyl group having 3 to 20 carbon atoms, an aryl group having 6 to 30 carbon atoms, a heteroaryl group having 3 to 30 carbon atoms, an alkoxy group, a hydroxy group, a halogen atom, a nitro group, a nitrile group, an NE¹E² group, a NE¹E²E²E³⁺ group, a carboxylic acid derivative group, a sulfonic acid derivative group, and the like. Among these, a hydrogen atom, an alkyl group having 2 to 20 carbon atoms, a cycloalkyl group having 3 to 20 carbon atoms, an alkoxy group, a halogen atom, and a nitro group are preferable, and a hydrogen atom, an alkyl group having 2 to 20 carbon atoms, and a cycloalkyl group having 3 to 20 carbon atoms are more preferable.

The transition metal complex preferably include one represented by the formula (10), more preferably one represented by the formula (10) in which each of R² and R³ is independently a tert-butyl group or a cycloalkyl group having 3 and 20 carbon atoms, and even more preferably one represented by the formula (10) in which R² and R³ are cycloalkyl groups having 5 to 8 carbon atoms.

One of these transition metal complexes may be used alone or two or more of these may be used in combination.

### (Alkene)

Examples of the alkene include, but are not particularly limited to, ethylene, propylene, isobutene, 1,3-butadiene, piperylene, 1-butene, 1-pentene, 1-hexene, 1-heptene, 1-octene, 1-nonene, 1-decene, and styrene. Among these, ethylene, propylene, 1,3-butadiene and styrene are preferable, and ethylene is more preferable.

One of these alkenes may be used alone, or two or more of these may be used in combination.

As for the state of the alkene, it may either be gaseous or liquid depending on the type thereof.

Generally, the type of an α,β-unsaturated carboxylic acid salt to be obtained varies depending on the type of an alkene used. For example, when ethylene is used, an acrylic salt is generally obtained. When propylene is used, a 2-butenoic salt is generally obtained. Likewise, the use of 1-butene generally yields a 2-ethylpropenoic salt.

The amount of the alkene to be used is preferably from 1 to 50 mol, more preferably 1.2 to 20 mol, even more preferably 1.5 to 15 mol, and particularly preferably 2 to 10 mol, per mol of the transition metal complex. When the amount of the alkene is not less than 1 mol, the reaction between the transition metal complex and the alkene can proceed efficiently. When the amount of the alkene is more than 1 mole (i.e., when the amount exceeds 1 mole per mol of the transition metal complex), the reaction in the step (2) may occur efficiently. Specifically, the alkene that has remained unreacted in the reaction of the step (1) induces a ligand exchange reaction with the complex of an α,β-unsaturated carboxylic acid salt produced in the step (2) described below. As a result, not only is the α,β-unsaturated carboxylic acid salt obtained, but the transition metal complex to which the alkene is coordinated can be regenerated to enable a continuous reaction. On the other hand, the use of the alkene in an amount of 50 mol or less is favorable in that the reaction pressure can be lowered.

### (Carbon Dioxide (CO₂))

CO₂ may be used is a gaseous or liquid form, or in its supercritical state. In the present invention, a gaseous mixture containing CO₂ applicable on an industrial scale can be used; however, if such a gaseous mixture is used, it is preferable to use one which is substantially free of carbon monoxide. In this context, "substantially free of carbon monoxide" means that the CO content of the gaseous mixture is not more than 100 ppm (0.01 % by volume) relative to 100 % by volume of the gaseous mixture.

The amount (A) of CO₂ to be used is 0.1 to 10 mol, preferably 0.1 to 3 mol, even more preferably 0.1 to 1.75 mol, even more preferably 0.1 to 1.2 mol, especially preferably 0.5 to 1.2 mol, and most preferably 0.5 to 1.0 mol, per mol of the transition metal complex. When the molar amount (A) of CO₂ is not less than 0.1 mol, the metal lactone compound can be produced efficiently. When the molar amount (A) of CO₂ is not more than 10 mol, it is possible to prevent CO₂ from remaining unreacted, thereby preventing or suppressing a side reaction between the base and CO₂ in the step (2) described below. The molar amount of CO₂ described above is significantly smaller than that in the above-mentioned Non-Patent Document 1 which uses 50 bar (50 atm) of CO₂. Thus, unlike Non-Patent Document 1, the present invention does not necessarily require high pressure conditions, and hence enables reduction of energy consumption and cost of manufacturing facilities.

Also, in one embodiment, the amount (A) of CO₂ to be used is preferably not more than 1 mol, more preferably 0.1 to 1 mol, and even more preferably 0.1 to 0.95 mol, per mol of the transition metal complex.

In conventional techniques such as the technique disclosed in Non-Patent Document 1 described above, since an alkoxide used for cleavage of the nickel-lactone reacts irreversibly with CO₂ to form an extremely stable carboxylic acid half-ester, the formation of such a carboxylic acid half-ester is suppressed by dividing the catalytic cycle into two regions, i.e., a CO₂-rich region and a CO₂-poor region. However, CO₂ still remains even in the CO₂-poor region, resulting in the formation of a carboxylic acid half-ester due to the reaction of an alkoxide with CO₂. Therefore, the alkoxide results in being consumed in the formation of the carboxylic acid half-ester.

In contrast, according to the above embodiment, the amount (A) of CO₂ to be used is not more than 1 mol per mol of the transition metal complex. That is, in the step (1), 1 mol of the transition metal complex reacts with 1 mol of CO₂, so that CO₂ is consumed entirely or almost entirely in the step (1). Therefore, the formation of a carboxylic acid half-ester can be prevented or suppressed in the step (2) because no or almost no CO₂ remains in the step (2) to be described below.

The present embodiment is particularly suitable for the continuous reaction described below because the formation of a carboxylic acid half-ester can be effectively prevented or suppressed.

### (Inert gas)

The reaction in the step (1) is preferably carried out under an atmosphere of inert gas.
The inert gas is not particularly limited, and may be a nitrogen gas, a noble gas (helium gas, argon gas, krypton gas, etc.), or the like. One of these inert gases may be used alone or two or more of these may be used in combination.

The inert gas is usually added together with CO₂ when CO₂ is gaseous. It may also be added with an alkene if the alkene is in a gaseous state.

The amount of inert gas used is preferably less than 50 % by volume, relative to the total volume of the inert gas, CO₂ and the alkene.

### (Solvent)

The reaction in the (1) is preferably carried out in a solvent. In one preferred embodiment, the reaction is preferably carried out by introducing the alkene and CO₂ into a solvent containing the transition metal complex to thereby bringing them into contact with the transition metal complex.

Examples of the solvent include, but are not particularly limited to, aromatic hydrocarbons such as benzene, toluene, and xylene; halogenated aromatic hydrocarbons such as chlorobenzene; ethers such as tetrahydrofuran (THF); dimethylformamide; and dimethyl sulfoxide. Among these, THF and toluene are preferable. One of these solvents may be used alone, or two or more of these may be used in combination.

### (Base)

The step (1) is preferably performed free of a base described below, which reacts with CO₂ to form a carboxylic acid half-ester.

However, for example, if the step (1) and the step (2) are repeated alternately as described below, the base may possibly get mixed in the system. In such a case, the amount of the base in the reaction system of the step (1) is preferably not more than 0.05 mol and more preferably not more than 0.02 mol, per mol of carbon dioxide.

### (Reaction)

There is no particular limitation with respect to the reaction mode in step (1), and the reaction may be carried out in any of a batchwise mode, a semi-batchwise mode, a continuous mode, or a combination of these.

In the case of a batchwise mode, it is preferable that the transition metal complex, an alkene and CO₂ are added to a solvent to perform a reaction. In the case of a semi-batchwise mode, it is preferable that the transition metal complex and an alkene are added to a solvent in advance, and then a reaction is performed while continuously introducing CO₂. In the case of a continuous mode, it is preferable that the transition metal complex is added to a solvent in advance, and a reaction is performed while continuously introducing an alkene and CO₂.

Further, in the case of performing the reaction in a continuous mode, the reaction can be performed in various manners such as a fixed bed process, a fluidized bed process, a moving bed process, a suspension bed process, or a process in which a liquid phase of a reaction mixture is withdrawn while supplying raw materials into a stirred tank reactor or a loop reactor. When the reaction is performed in a liquid phase in a stirred tank reactor in a batchwise mode or a continuous mode using gaseous alkene and CO₂, the alkene and CO₂ may be supplied to either one of the gaseous phase and the liquid phase in the reactor, or to both of the gaseous phase and the liquid phase in the reactor.

As for the reactor, any conventional reactors can be appropriately used depending on the reaction mode employed.

The reaction temperature in the step (1) is preferably 50 to 250 °C, and more preferably 80 to 200 °C.

In general, the reaction pressure in the step (1) is preferably not higher than 50 atm, more preferably not higher than 10 atm, even more preferably 2 to 10 atm, and particularly preferably 3 to 10 atm, in terms of absolute pressure.

### (Metal Lactone Compound)

The metal lactone compound obtained by the reaction of the step (1) is represented by formula (1) below.

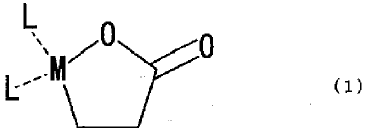

In the formula (1), M is a transition metal, and each L is independently a monodentate ligand, or two L together form a bidentate ligand. The transition metal and the ligand (monodentate or bidentate ligand) are as listed above.

In one embodiment, the metal lactone compound is preferably one represented by formula (2) below.

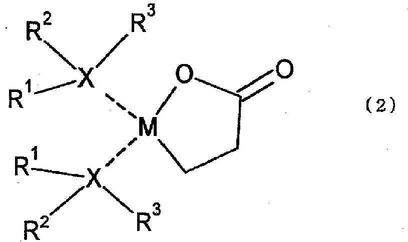

In the formula (2), M is a transition metal, each X is independently a nitrogen atom or a phosphorus atom, each R¹ is independently an aliphatic hydrocarbon group, an aromatic hydrocarbon group, a heteroaromatic group, or a nitrogen-containing group, each of R² and R³ is independently an aliphatic hydrocarbon group, an aromatic hydrocarbon group, or a heteroaromatic group, with the proviso that two R¹ may be bonded to each other to form a ring structure, and R¹, R², and R³, which are bonded to the same X atom, may be bonded together to form a ring structure.

The above-mentioned M, X, R¹, R², R³ are the same as explained above.

In one embodiment, the metal lactone compound is more preferably one represented by formula (3) below.

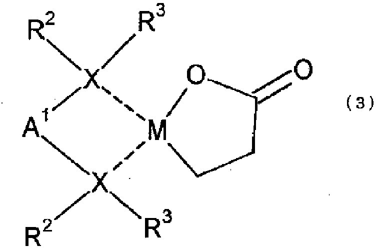

In the formula (3), M is a transition metal, each X is independently a nitrogen atom or a phosphorus atom, each of R² and R³ is independently an aliphatic hydrocarbon group, an aromatic hydrocarbon group, or a heteroaromatic group, A¹ is a single bond, a divalent aliphatic hydrocarbon group, a divalent aromatic hydrocarbon group, a divalent heteroaromatic group or a nitrogen-containing group, with the proviso that A¹, R², and R³, which are bonded to the same X atom, may be bonded together to form a ring structure.

As for M, X, R², R³, and A, the same applies as described above.

In the formula (3) above, at least one of the X atoms is preferably a nitrogen atom. In this case, it is preferable that a heteroaromatic ring is formed by mutual bond between A¹, R², and R³, which are bonded to the nitrogen atom. Examples of the heteroaromatic ring include the same as those described above for the formula (7).

In the formula (3), it is preferable that at least one of the X atoms is a phosphorus atom, and at least one of R² and R³, which are bonded to the phosphorus atom, is an aliphatic hydrocarbon group having 3 or more carbon atoms, an aromatic hydrocarbon group having 6 or more carbon atoms, or a heteroaromatic group having 3 or more carbon atoms. Examples of the aliphatic hydrocarbon group having 3 or more carbon atoms, the aromatic hydrocarbon group having 6 or more carbon atoms, and the heteroaromatic group having 3 or more carbon atoms include the same as those mentioned above for the formula (7).

In one embodiment, the metal lactone compound is more preferably at least one selected from the group consisting of the compounds represented by the following formulae (4) to (6).

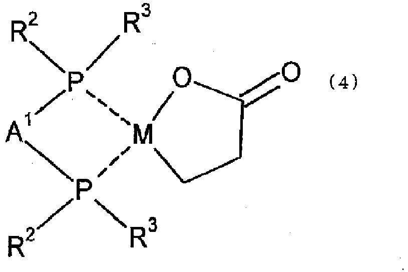

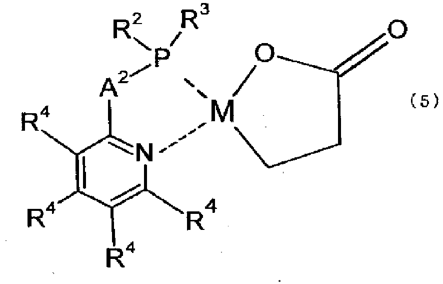

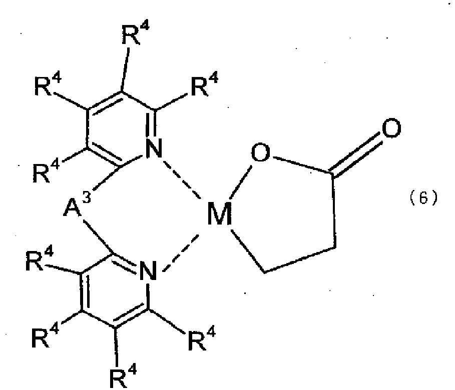

In the formulae (4) to (6), M is a transition metal, each of R² and R³ is independently an aliphatic hydrocarbon group, an aromatic hydrocarbon group, or a heteroaromatic group, each of A¹, A² and A³ is a single bond, a divalent aliphatic hydrocarbon group, a divalent aromatic hydrocarbon group, a divalent heteroaromatic group or a nitrogen-containing group, B is a single bond, a divalent aliphatic hydrocarbon group, a divalent aromatic hydrocarbon group, a divalent heteroaromatic group or a nitrogen-containing group, C is a single bond, a divalent aliphatic hydrocarbon group, a divalent aromatic hydrocarbon group, a divalent heteroaromatic group or a nitrogen-containing group, and each R⁴ is independently a hydrogen atom, an aliphatic hydrocarbon group, an aromatic hydrocarbon group, or a heteroaromatic group.

As for M, R², R³, A¹, A², A³, and R⁴, the same applies as described above.

Among these, the metal lactone compound is preferably a compound represented by the formula (5).

One type of the metal lactone compound may be formed alone, or two or more types of the metal lactone compound may be formed in the formed of a mixture thereof.

### [Step (2)]

In the step (2), a base is allowed to act on the metal lactone compound.

### (Metal Lactone Compound)

As for the metal lactone compound, the same applies as described above, and further explanation is omitted.

### (Base)

The base is not particularly limited as long as it is capable of advancing the β-hydrogen elimination reaction of the metal lactone and the subsequent reductive dehydrogenation, but sodium tert-butoxide (t-BuONa), sodium bis(trimethylsilyl)amide (NaHMDS), lithium iodide (LiI), ammonia (NH₃), and phenoxide (PhONa) can be listed as examples.

One of these bases may be used alone, or two or more of these may be used in combination. For example, it is preferable that lithium iodide (LiI) and ammonia (NH₃) is used in combination.

The amount of the base to be used is preferably 0.1 to 10.0 mol, more preferably 0.3 to 5.0 mol, and even more preferably 0.5 to 1.5 mol, per mol of the transition metal complex.

Further, it is preferable that the molar amount (B) of the base per mol of the transition metal complex in the step (2) is not more than the molar amount (A) of the carbon dioxide. Specifically, the ratio (B/A) of the molar amount of the base (B) to the molar amount (A) of the carbon dioxide is preferably not more than 1, more preferably 0.8 to 1, even more preferably 0.9 to 1, and particularly preferably 0.95 to 1. The ratio B/A of not more than 1 is favorable in that, when a continuous reaction described below is performed, the formation of the carboxylic acid half-ester can be prevented or suppressed in the second and subsequent runs of the step (1). More specifically, the base is consumed entirely or almost entirely by reaction with the nickel-lactone complex obtained in the step (1). No or almost no base remains after the reaction in the step (2), so that its reaction with CO₂, which is added in the subsequent run of the step (1), can be prevented or suppressed.

In one preferred embodiment, it is preferable that the molar amount (A) of the carbon dioxide is not more than 1 mol per mol of the transition metal complex in the step (1), and the molar amount (B) of the base is not more than 1 mol per mole of the transition metal complex in the step (2), and it is more preferable that the molar amount (A) of the carbon dioxide is 0.1 to 0.95 mol, and the molar amount (B) of the base is 0.1 to 0.95 mol.

### (Lewis Acid)

In the step (2), a Lewis acid may be used. The use of a Lewis acid can increase the reaction rate and/or moderate the reaction conditions (lower the reaction temperature) in the step (2).

As for the Lewis acids, there are no particular limitations, but LiI, NaI, AlCl₃, Al(OEt)₂Cl, ZnO, SiO₂, Al₂O₃ and the like can be listed as examples. Of these, LiI, Nal and AlCl₃ are preferable, and Lil and Nal are more preferable.

One of the Lewis acids described above may be used alone, or two or more thereof may be used in combination.

The amount of the Lewis acid used is preferably 0.2 to 20 mol, more preferably 0.5 to 15 mol, and even more preferably 1 to 10 mol, per mol of the transition metal complex. When the amount of the Lewis acid used is not less than 0.2 mol, the cleavage of the lactone ring proceeds efficiently whereby the production efficiency of an α,β-unsaturated carboxylic acid can be favorably increased. When the amount of the Lewis acid used is not more than 20 mol, the amount of the Lewis acid used can be suppressed, which is economically favorable.

When a Lewis acid is used, the molar ratio of the base to the Lewis acid (number of moles of base / number of moles of Lewis acid) is preferably 0.2 to 5, more preferably 0.5 to 3, and even more preferably 0.8 to 2. The molar ratio of not less than 0.5 is favorable in that hydrogen is eliminated from a hydride intermediate produced by cleavage of the lactone ring and the subsequent reductive elimination proceeds efficiently, thereby enabling an α,β-unsaturated carboxylic acid to be produced with higher efficiency. On the other hand, when the molar ratio is not more than 5, the relative amount of the base can be reduced, which is economically favorable.

### (Alkene)

The step (2) is preferably performed in the presence of an alkene. The presence of the alkene in the step (2) is preferred because, as described below, not only is the α,β-unsaturated carboxylic acid salt obtained, but the transition metal complex is regenerated as well.

Specific examples of the alkene include those described above.

The alkene may be one left over from the step (1), one newly introduced in the step (2), or a combination thereof. In one embodiment, the alkene is preferred to be one left over from the step (1) from the perspective of simplicity of the process.

The amount of the alkene used in the step (2) is preferably 0.1 to 50 mol, more preferably 0.5 to 20 mol, even more preferably 1.0 to 15 mol, and particularly preferably 1.5 to 10 mol, per mol of the transition metal complex.

### (Action)

In the step (2), a base is allowed to act on the metal lactone compound to cleave the lactone ring of the metal lactone compound to yield a transition metal complex to which an α,β-unsaturated carboxylic acid salt is coordinated. More specifically, the metal lactone compound first reacts with the base to have the lactone ring of the metal lactone compound cleaved by β-hydrogen elimination, and the subsequent reductive dehydrogenation yields a transition metal complex to which an α,β-unsaturated carboxylic acid salt is coordinated.

Then, the transition metal complex to which the α,β-unsaturated carboxylic acid salt is coordinated is subjected to a ligand exchange reaction with an arbitrary ligand (e.g., an alkene or the like), whereby an α,β-unsaturated carboxylic acid salt can be obtained. More specifically, the transition metal complex to which the α,β-unsaturated carboxylic acid salt is coordinated is allowed to undergo a ligand exchange reaction with an arbitrary ligand to thereby obtain an α,β-unsaturated carboxylic acid salt as well as a transition metal complex to which the arbitrary ligand is coordinated. The finally obtained transition metal complex to which the arbitrary ligand is coordinated as such can react with CO₂ to obtain a metal lactone compound, thereby enabling the production to be performed via a continuous reaction. As mentioned above, it is preferable that the arbitrary ligand is an alkene.

The reaction temperature in the step (2) is preferably 20 to 250 °C, and more preferably 50 to 200 °C.

The reaction pressure in the step (2) is preferably not higher than 50 atm, more preferably not higher than 10 atm, even more preferably 2 to 10 atm, and particularly preferably 3 to 10 atm, in terms of absolute pressure.

### [Continuous Reaction]

The present invention, in one embodiment thereof, provides a method for producing an α,β-unsaturated carboxylic acid salt, in which the steps (1) and (2) are alternately repeated.

In the conventional method, an excess amount of CO₂ present in the system reacts preferentially with a base, resulting in the formation of a carboxylic acid half-ester, so that the reaction cannot be performed repeatedly in an efficient manner. In contrast, according to the embodiment described above, such a side reaction can be prevented and the reaction can be performed repeatedly.

### Examples

Hereinbelow, the present invention will be described with reference to Examples and Comparative Examples which, however, should not be construed as limiting the present invention.

### [Example 1]

### (1) Synthesis of MAP ligand

2-(methylamino)pyridine (0.459 g, 4.16 mmol) was dissolved in 15 mL of toluene, and the resulting solution was cooled to -35 °C. To the cooled solution was gradually added dropwise n-butyl lithium (1.6 M, 2.75 mL, 4.37 mmol), and the resulting was stirred for 3 hours at room temperature. The resulting was cooled again to -35 °C, whereafter cyclohexylphosphine (0.967 g, 4.16 mmol) was dropwise added, and the obtained mixture was stirred for 8 hours at 80 °C. Then, toluene was distilled off from the mixture in vacuo, and the resulting was dissolved in pentane, followed by filtration of the obtained solution over Celite. Hexane was distilled off from the residue in vacuo, to thereby obtain (dihexylphosphino)-2-(methylamino)pyridine (MAP) (870 mg, 69 %) represented by the following formula (5) as a desired product.

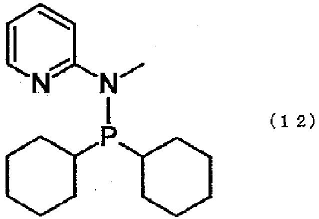

### (2) Synthesis of (MAP)Ni(COD) metal complex as transition metal complex

A tetrahydrofuran (THF) solution of (dihexylphosphino)-2-methylaminopyridine (MAP) (64.6 mg, 0.212 mmol) was gradually added dropwise to a THF solution of bis(1,5-cyclooctadiene)nickel (Ni(COD)2) (58.4 mg, 0.212 mmol). The resulting mixture was stirred at room temperature for 3 hours, and then the THF was distilled off in vacuo. As a result, a transition metal complex ((MAP)Ni(COD)) represented by the following formula was obtained.

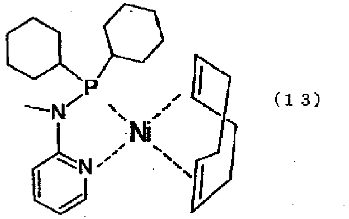

### (3) Step (1)

A transition metal complex, (MAP)Ni(COD), (0.2 mmol, 94.3 mmol) was suspended in THF (10 mL) in a nitrogen atmosphere. The resulting suspension was placed in Series 5500 Compact Microreactor (volume: 25 mL, manufactured by Parr Instrument Company) which is a reaction vessel. This reaction vessel was connected to a heater and Series 4848 Reactor Controller (manufactured by Parr Instrument Company), which is a reactor controller. Thereafter, 4 mol of ethylene and subsequently 1 mol of carbon dioxide were introduced into the reaction vessel (internal pressure: about 3 atm) per mol of the transition metal complex. The resulting solution in the reaction vessel was stirred at 80 °C for 3 hours. As a result, a metal lactone compound represented by the following formula was obtained.

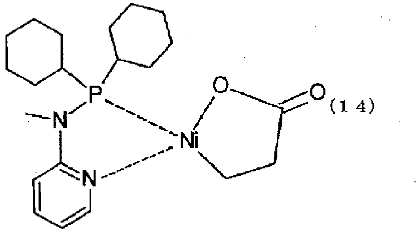

### (4) Step (2)

Sodium tert-butoxide (tert-BuONa) (19.2 mg, 0.2 mmol) dissolved in THF was added to the reaction solution obtained in step (1) in the reaction vessel using a metering pump. After the reaction, the reaction vessel was cooled to room temperature to thereby obtain an acrylic acid salt.

### (5-1) Measurement of CO₂ conversion

To the resulting reaction solution were added 20 mL of heavy water (D₂O), sorbic acid (2,4-hexadienoic acid) (50 mg), sodium hydroxide (added to convert the sorbic acid into a sodium salt because sorbic acid is not soluble in heavy water) (100 mg), and the resulting mixture was stirred at room temperature for 10 minutes. Thereafter, 50 mL of diethyl ether was added, and the resulting was allowed to separate into an ether layer and a D₂O layer. The amount of sodium acrylate generated was calculated based on the results of ¹H-NMR analysis of the D₂O layer. More specifically, from the results of ¹H - NMR analysis, an integral ratio of the proton at the β position of sodium acrylate relative to the proton at the β position of sorbic acid as a standard substance was calculated to determine the amount of sodium acrylate generated (CO₂ conversion = amount of sodium acrylate obtained/amount of CO₂ fed). The CO₂ conversion was evaluated according to the following criteria. The results are shown in Table 1.
A: CO₂ conversion is 90 % or more.
B : CO₂ conversion is not less than 75 % but less than 90 %.
C : CO₂ conversion is not less than 60 % but less than 75 %.
D : CO₂ conversion is not less than 20 % but less than 60 %.
E : CO₂ conversion is less than 20 %.

### (5-2) Measurement of Carboxylic Acid Half-Ester Content

The carboxylic acid half-ester content was measured by NMR. The carboxylic acid half-ester content was evaluated according to the following criteria. The results are shown in Table 1.
A: Carboxylic acid half-ester content is 0 %.
B : Carboxylic acid half-ester content is more than 0 % but less than 10 %.
C : Carboxylic acid half-ester content is not less than 10 % but less than 15 %.
D : Carboxylic acid half-ester content is not less than 15 % but less than 45 %.
E : Carboxylic acid half-ester content is not less than 45 % but less than 70 %.
F : Carboxylic acid half-ester content is not less than 70 %.

### [Example 2]

An acrylic acid salt was produced in the same manner as in Example 1, except that, in the step (1), the amount of ethylene added per mol of the transition metal complex was changed to 2.5 mol and the pressure (internal pressure of the reactor) was changed to 4 atm.

The CO₂ conversion and the carboxylic acid half-ester content were determined in the same manner as in Example 1. The results are shown in Table 1.

### [Example 3]

An acrylic acid salt was produced in the same manner as in Example 2, except that, in the step (1), the reaction temperature was changed to 50 °C.

The CO₂ conversion and the carboxylic acid half-ester content were determined in the same manner as in Example 1. The results are shown in Table 1.

### [Example 4]

An acrylic acid salt was produced in the same manner as in Example 2, except that, in the step (1), the reaction temperature was changed to 25 °C.
The CO₂ conversion and the carboxylic acid half-ester content were determined in the same manner as in Example 1. The results are shown in Table 1.

### [Example 5]

An acrylic acid salt was produced in the same manner as in Example 2, except that, in the step (1), the pressure was changed to 2 atm.

The CO₂ conversion and the carboxylic acid half-ester content were determined in the same manner as in Example 1. The results are shown in Table 1.

### [Example 6]

An acrylic acid salt was produced in the same manner as in Example 2, except that, in the step (1), the amount of CO₂ added per mol of the transition metal complex was changed to 0.2 mol and the amount of the base added per mol of the transition metal complex was changed to 0.2 mol.

### [Example 7]

An acrylic acid salt was produced in the same manner as in Example 2, except that, in the step (1), the amount of CO₂ added per mol of the transition metal complex was changed to 0.5 mol and the amount of the base added per mol of the transition metal complex was changed to 0.5 mol.

### [Example 8]

An acrylic acid salt was produced in the same manner as in Example 2, except that, in the step (1), the amount of CO₂ added per mol of the transition metal complex was changed to 0.8 mol and the amount of the base added per mol of the transition metal complex was changed to 0.8 mol.

### [Example 9]

An acrylic acid salt was produced in the same manner as in Example 2, except that, in the step (1), the amount of CO₂ added per mol of the transition metal complex was changed to 1.25 mol.

The CO₂ conversion and the carboxylic acid half-ester content were determined in the same manner as in Example 1. The results are shown in Table 1.

### [Example 10]

An acrylic acid salt was produced in the same manner as in Example 9, except that, in the step (1), the pressure was changed to 2.8 atm.

The CO₂ conversion and the carboxylic acid half-ester content were determined in the same manner as in Example 1. The results are shown in Table 1.

### [Example 11]

An acrylic acid salt was produced in the same manner as in Example 9, except that, in the step (1), the pressure was changed to 1.3 atm.

The CO₂ conversion and the carboxylic acid half-ester content were determined in the same manner as in Example 1. The results are shown in Table 1.

### [Example 12]

An acrylic acid salt was produced in the same manner as in Example 11, except that, in the step (1), the amount of CO₂ added per mol of the transition metal complex was changed to 2 mol.

The CO₂ conversion and the carboxylic acid half-ester content were determined in the same manner as in Example 1. The results are shown in Table 1.

### [Example 13]

An acrylic acid salt was produced in the same manner as in Example 11, except that, in the step (1), the amount of CO₂ added per mol of the transition metal complex was changed to 4 mol.

The CO₂ conversion and the carboxylic acid half-ester content were determined in the same manner as in Example 1. The results are shown in Table 1.

### [Example 14]

An acrylic acid salt was produced in the same manner as in Example 2, except that the transition metal complex used in the step (1) was changed to (dcpe)Ni(COD) represented by the following formula, and the reaction time in the step (1) was changed to 24 hours.

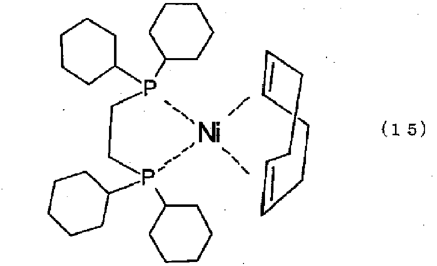

The CO₂ conversion and the carboxylic acid half-ester content were determined in the same manner as in Example 1. The results are shown in Table 1.

### [Example 15]

An acrylic acid salt was produced in the same manner as in Example 14, except that, in the step (1), the amount of CO₂ added per mol of the transition metal complex was changed to 1.25 mol.

The CO₂ conversion and the carboxylic acid half-ester content were determined in the same manner as in Example 1. The results are shown in Table 1.

### [Comparative Example 1]

An acrylic acid salt was produced in the same manner as in Example 1, except that, in the step (1), sodium tert-butoxide (t-BuONa) was blended together with the transition metal complex and the step (2) was omitted.

The CO₂ conversion and the carboxylic acid half-ester content were determined in the same manner as in Example 1. The results are shown in Table 1.

**[Table 1]**

| | Step (1) | | | | | | | Step (2) | | Evaluation of results |
|---|---|---|---|---|---|---|---|---|---|---|
| | Transition metal complex | Alkene (mol) * | Carbon dioxide (mol)* | Base (mol)* | Temp. (°C) | Pressure (atm) | Time (hr.) | Base (mol)* | CO₂ conversion | Carboxylic acid half-ester content |
| Ex.1 | (MAP)Ni(COD) | 4 | 1 | 0 | 80 | 3 | 3 | 1 | A | A |
| Ex.2 | (MAP)Ni(COD) | 2.5 | 1 | 0 | 80 | 4 | 3 | 1 | A | A |
| Ex.3 | (MAP)Ni(COD) | 2.5 | 1 | 0 | 50 | 4 | 3 | 1 | A | A |
| Ex.4 | (MAP)Ni(COD) | 2.5 | 1 | 0 | 25 | 4 | 3 | 1 | A | A |
| Ex.5 | (MAP)Ni(COD) | 2.5 | 1 | 0 | 80 | 2 | 3 | 1 | A | A |
| Ex.6 | (MAP)Ni(COD) | 2.5 | 0.2 | 0 | 80 | 4 | 3 | 0.2 | A | A |
| Ex.7 | (MAP)Ni(COD) | 2.5 | 0.5 | 0 | 80 | 4 | 3 | 0.5 | A | A |
| Ex.8 | (MAP)Ni(COD) | 2.5 | 0.8 | 0 | 80 | 4 | 3 | 0.8 | A | A |
| Ex.9 | (MAP)Ni(COD) | 2.5 | 1.25 | 0 | 80 | 4 | 3 | 1 | B | B |
| Ex.10 | (MAP)Ni(COD) | 2.5 | 1.25 | 0 | 80 | 2.8 | 3 | 1 | B | C |
| Ex.11 | (MAP)Ni(COD) | 2.5 | 1.25 | 0 | 80 | 1.3 | 3 | 1 | C | C |
| Ex.12 | (MAP)Ni(COD) | 2.5 | 2 | 0 | 80 | 1.3 | 3 | 1 | D | D |
| Ex.13 | (MAP)Ni(COD) | 2.5 | 4 | 0 | 80 | 1.3 | 3 | 1 | D | E |
| Ex.14 | (dcpe)Ni(COD) | 2.5 | 1 | 0 | 80 | 4 | 24 | 1 | B | A |
| Ex.15 | (dcpe)Ni(COD) | 2.5 | 1.25 | 0 | 80 | 4 | 24 | 1 | C | C |
| Comp.Ex.1 | (MAP)Ni(COD) | 4 | 1 | 1 | 80 | 3 | 3 | 0 | E | F |

| | | | | | | | | | | |
|---|---|---|---|---|---|---|---|---|---|---|
| * The values with "mol" mean amounts per mol of the transition metal complex. | | | | | | | | | | |

The results in Table 1 show that acrylic acid salts could be produced efficiently with less CO₂ in Examples 1 to 12. Further, it can be seen that the formation of a carboxylic acid half-ester as byproduct could be prevented as compared to Comparative Example 1.

### [Example 16]

After adding tert-BuONa in the step (2) of Example 1, a sequence of the step (1) and the step (2) was repeated one more time. The 2nd run of the steps was implemented in the same manner as in Example 1, except that the transition metal complex, (MAP)Ni(COD), was not added.

The CO₂ conversion and the carboxylic acid half-ester content were determined in the same manner as in Example 1. As a result, the CO₂ conversion was "A" and the carboxylic acid half-ester content was "A".

From the results of Example 13, it is considered that the addition of tert-BuONa in the 1st run of the step (2) allowed an α,β-unsaturated carboxylic acid salt (sodium acrylate) to be released from the metal lactone compound, which is simultaneously accompanied by ligand exchange with COD (1,5-cyclooctadiene) or ethylene remaining in the system, thereby regenerating (MAP) Ni(COD) or (MAP)Ni(ethylene). This is considered to be the reason why a further α,β-unsaturated carboxylic acid salt (sodium acrylate) could be obtained in the 2nd run by going through the steps (1) and (2) in the same manner.

### [Example 17]

After adding tert-BuONa in the step (2) of Example 8, a sequence of the step (1) and the step (2) was repeated three more times. Each of the three-times repeated sequence of the steps was implemented in the same manner as in Example 8, except that the transition metal complex, (MAP)Ni(COD), was not added, and the amount of ethylene used in the step (1) was changed to 0.8 mol per mol of the transition metal complex (per 1 mol of the transition metal complex used in the 1st run).

The CO₂ conversion and the carboxylic acid half-ester content were determined in the same manner as in Example 1. The results are shown in Table 2.

**[Table 2]**

| | Run No. | Step (1) | | | | | | | Step (2) | CO₂ conversion | |
|---|---|---|---|---|---|---|---|---|---|---|---|
| | | Transition metal complex | Alkene (mol)* | Carbon dioxide (mol)* | Base (mol)* | Temp. | Pressure (atm) | Time | Base (mol) | CO₂ conversion | Carboxylic acid half-ester content |
| Ex.17 | 1 | (MAP)Ni(COD) | 2.5 | 0.8 | 0 | 80 | 4 | 3 | 0.8 | A | A |
| | 2 | (MAP)Ni(COD) | 0.8 | 0.8 | 0 | 80 | 4 | 3 | 0.8 | A | A |
| | 3 | (MAP)Ni(COD) | 0.8 | 0.8 | 0 | 80 | 4 | 3 | 0.8 | A | A |
| | 4 | (MAP)Ni(COD) | 0.8 | 0.8 | 0 | 80 | 4 | 3 | 0.8 | A | A |

The results in Table 2 show that all of the added CO₂ had been consumed and the reaction efficiency was high. The same conclusion can also be derivable from the fact that the carboxylic acid half-ester content was "A" even after a total of four runs of continuous reactions.

## Claims

1. A method for producing an α,β-unsaturated carboxylic acid salt, comprising:
a step (1) of reacting a transition metal complex, an alkene and carbon dioxide to obtain a metal lactone compound represented by formula (1): 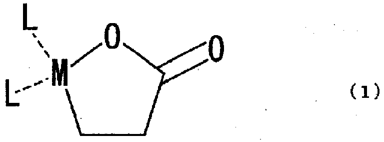 wherein:
M is a transition metal,
each L is independently a monodentate ligand, or two L together form a bidentate ligand; and
a step (2) of allowing a base to act on the metal lactone compound,
wherein a molar amount (A) of the carbon dioxide per mol of the transition metal complex in the step (1) is 0.1 to 10 mol.

2. The method according to claim 1, wherein the metal lactone compound is represented by formula (2): 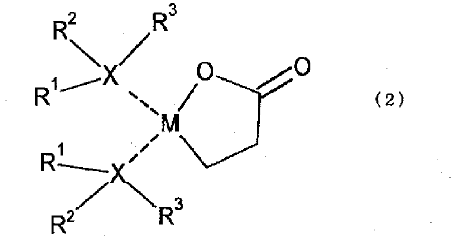 wherein:
M is a transition metal,
each X is independently a nitrogen atom or a phosphorus atom,
each R¹ is independently an aliphatic hydrocarbon group, an aromatic hydrocarbon group, a heteroaromatic group, or a nitrogen-containing group, and
each of R² and R³ is independently an aliphatic hydrocarbon group, an aromatic hydrocarbon group, or a heteroaromatic group,
with the proviso that two R¹ are or are not bonded to each other to form a ring structure, and
R¹, R², and R³, which are bonded to the same X atom, are or are not bonded together to form a ring structure.

3. The method according to claim 2, wherein the metal lactone compound is represented by formula (3): 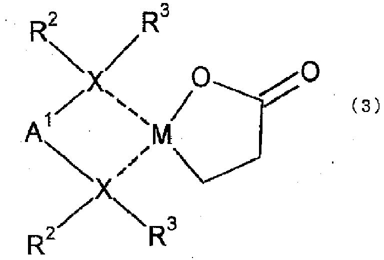 wherein:
M is a transition metal,
each X is independently a nitrogen atom or a phosphorus atom,
each of R² and R³ is independently an aliphatic hydrocarbon group, an aromatic hydrocarbon group, or a heteroaromatic group, and
A¹ is a single bond, a divalent aliphatic hydrocarbon group, a divalent aromatic hydrocarbon group, a divalent heteroaromatic group or a nitrogen-containing group,
with the proviso that A¹, R², and R³, which are bonded to the same X atom, are or are not bonded together to form a ring structure.

4. The method according to claim 3, wherein at least one of the X is a nitrogen atom, and
A¹, R², and R³, which are bonded to the nitrogen atom, are bonded together to form a heteroaromatic ring.

5. The method according to claim 3 or 4, wherein at least one of the X is a phosphorus atom, and
at least one of R² and R³, which are bonded to the phosphorus atom, is an aliphatic hydrocarbon group having 3 or more carbon atoms, an aromatic hydrocarbon group having 6 or more carbon atoms, or a heteroaromatic group having 3 or more carbon atoms.

6. The method according to any one of claims 3 to 5, wherein the metal lactone compound is at least one compound represented by formulae (4) to (6): 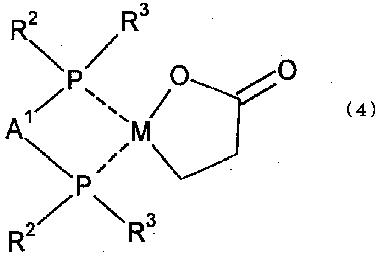 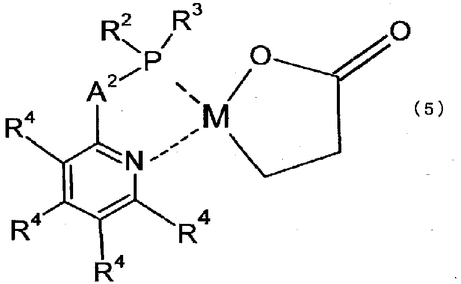 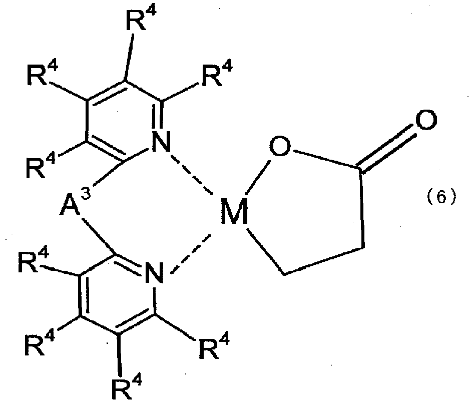 wherein:
M is a transition metal,
each of R² and R³ is independently an aliphatic hydrocarbon group, an aromatic hydrocarbon group, or a heteroaromatic group,
A¹ is a single bond, a divalent aliphatic hydrocarbon group, a divalent aromatic hydrocarbon group, a divalent heteroaromatic group or a nitrogen-containing group,
A² is a single bond, a divalent aliphatic hydrocarbon group, a divalent aromatic hydrocarbon group, a divalent heteroaromatic group or a nitrogen-containing group,
A³ is a single bond, a divalent aliphatic hydrocarbon group, a divalent aromatic hydrocarbon group, a divalent heteroaromatic group or a nitrogen-containing group, and
each R⁴ is independently a hydrogen atom, an aliphatic hydrocarbon group, an aromatic hydrocarbon group, or a heteroaromatic group.

7. The method according to any one of claims 1 to 6, wherein a molar amount (B) of the base per mol of the transition metal complex in the step (2) is not more than the molar amount (A) of the carbon dioxide.

8. The method according to any one of claims 1 to 7, wherein the reaction in the step (1) is carried out at a pressure of 10 atm or less.

9. The method according to any one of claims 1 to 8, wherein the step (1) and the step (2) are alternately repeated.
